# EUROPÄISCHE PATENTANMELDUNG

(11) **EP 1 169 996 A1**
(43) Veröffentlichungstag der Anmeldung: **09.01.2002**
(21) Anmeldenummer: 00114527.5
(22) Anmeldetag: 06.07.2000
(51) Int. Cl.: A61K 6/083, C07F 9/38

(54) **Phosphonsäuren enthaltendes Dentalmaterial**

(71) Anmelder: ERNST MÜHLBAUER KG, 22547 Hamburg (DE)
(72) Erfinder: Mühlbauer, Wolfgang, Dr., 22609 Hamburg (DE); Neffgen, Stephan, Dr., 22459 Hamburg (DE); Erdmann, Christoph, Dr., 20255 Hamburg (DE)
(74) Vertreter: Glawe, Delfs, Moll & Partner

(57) **Zusammenfassung**

Gegenstand der Erfindung sind Dentalmaterialien, die hydrolysestabile Phosphonsäuren mit ethylenisch ungesättigten Doppelbindungen enthalten und die sich besonders als Haftvermittler eignen.

## Beschreibung

Die Erfindung betrifft Dentalmaterialien, insbesondere Haftvermittler oder polymerisierbare Zemente wie bspw. Compomere oder Glasionomerzemente mit polymerisierbaren Säuren, wie sie grundsätzlich aus DE-A-3536076 und DE-A-3536077 bekannt sind.

Dentalmaterialien mit polymerisierbaren Phosphorsäuren als Comonomere sind bekannt, sie enthalten üblicherweise Phosphorsäure und Acrylate oder Methacrylate. Aus DE-A-19647140 ist es bspw. bekannt, ein Hydroxyalkylacrylat bzw. -methacrylat mit Phosphat zu verestern. Nachteilig an diesen Substanzen ist die geringe Hydrolysestabilität, da die Esterbindungen zwischen Phosphat und Alkylkette sowie zwischen Methacrylat und Alkylkette leicht hydrolytisch gespalten werden können. Dies vermindert die Lagerstabilität des Dentalmaterials und führt unter den Bedingungen in der Mundhöhle zu einer reduzierten Dauerhaltbarkeit im Zahn.

Aus DE-A-19746708 ist es bekannt, Phosphonate über einen Spacer an die Methylgruppe eines Methacrylatesters zu binden. Bekannt sind ferner Phosphinoxide, die über eine Urethangruppe mit Methacrylaten oder Styrol verknüpft sind (J. Smid et al., Journal of Polymer Science, Part A Polymer Chemistry, 31, 239 - 247 (1993)) sowie die Verknüpfung von Phosphonsäuren mit Methacrylaten über einen Ester (DE-A-19918974). Der Erfindung liegt die Aufgabe zugrunde, Dentalmaterialien der eingangs genannten Art zu schaffen, die gute Hafteigenschaften und eine hohe Hydrolysestabilität aufweisen.

Die Erfindung löst diese Aufgabe durch die Merkmale des Hauptanspruchs. Dementsprechend enthalten die Dentalmaterialien Phosphonsäuren der nachfolgend angegebenen Struktur bzw. Salze dieser Säuren: 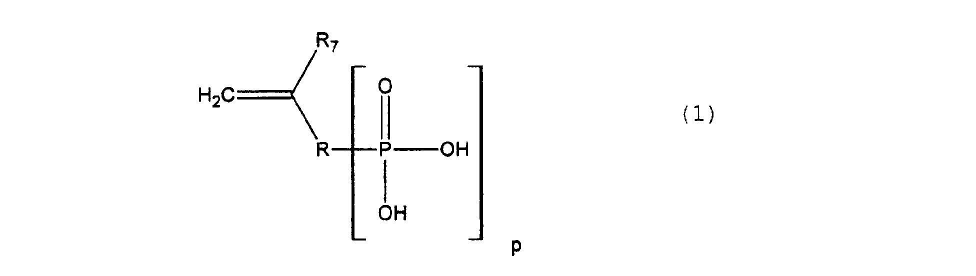 wobei R, R₇ und p unabhängig voneinander bedeuten:
entweder:
a) R: Alkylgruppe oder Alkylengruppe mit mindestens 6 C-Atomen oder Arylgruppe,
   R₇: H, Methyl, Ethyl, Propyl, Isopropyl oder Butyl, p gleich 1 oder 2;
   oder:
b) R: -CO-NR₁-R₈-
   mit R₁ gleich H, Alkyl oder Aryl,
   mit R₈ gleich Aryl oder CₙH₂ₙ
   wobei 4 ≤ n ≤ 18 ist,
   oder R₈ gleich CₙH₂ₙ-Si(R₅)₂-[O-Si(R₅)₂]ₘ-CₙH₂ₙ-
   wobei 3 ≤ n ≤ 12,
   1 ≤ m ≤ 10, und
   R₅ gleich Methyl, Ethyl oder Phenyl ist,
   oder R₈ gleich CₙH₂ₙ-COONH-CₙH₂ₙ
   wobei 4 ≤ n ≤ 12 ist, und wobei R₈ Ether- oder weitere Urethangruppen aufweisen kann,
   R₇ und p wie in a) definiert sind;
   oder:
c) R: 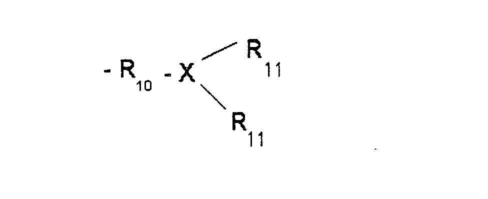 mit R₁₀ ausgewählt aus der Gruppe bestehend aus Arylgruppen, Alkylgruppen mit mindestens 3 C-Atomen, oder Polyethergruppen mit 1 bis 10 Polyethereinheiten, mit R₁₁ gleich oder verschieden ausgewählt aus der Gruppe bestehend aus Alkylgruppen und Arylgruppen, mit X gleich N, B oder CH,
   R₇: COOR₉, CONHR₉, H oder Phenyl mit R₉ ausgewählt aus der Gruppe bestehend aus Methyl, Ethyl, Propyl, Isopropyl und Butyl,
   p gleich 2.

Der Erfindung liegt die überraschende Erkenntnis zugrunde, daß Moleküle mit einer verhältnismäßig langkettigen Brücke zwischen Phosphonsäuregruppe und reaktiver Doppelbindung, wie sie im Anspruch definiert ist, eine hohe Hydrolysestabilität und gleichzeitig verbesserte Hafteigenschaften aufweisen.

Bevorzugte Ausgestaltungen der Erfindung sind in den Unteransprüchen angegeben.

Eine bevorzugte Ausführungsform der Erfindung hat die nachfolgend angegebene Struktur:

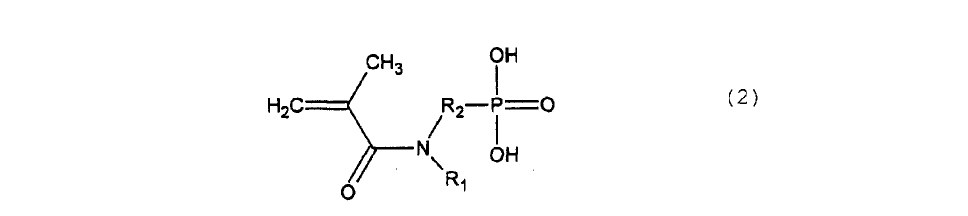

R₁ ist Wasserstoff, eine Alkyl- oder Arylgruppe. Bevorzugt wird R₁ ausgewählt aus der Gruppe bestehend aus Wasserstoff, Methyl, Ethyl und Isopropyl. R₂ ist eine Alkylgruppe mit 4 bis 18 C-Atomen, die unverzweigt oder verzweigt sein kann. Bevorzugt enthält sie 6 bis 12 C-Atome. Im Rahmen der Erfindung kann R₂ auch als Alkylkette ausgebildet sein, die durch Ethergruppen oder weitere Urethangruppen unterbrochen ist.

Bei einer weiteren bevorzugten Ausführungsform haben die Phosphonsäuren folgende Struktur:

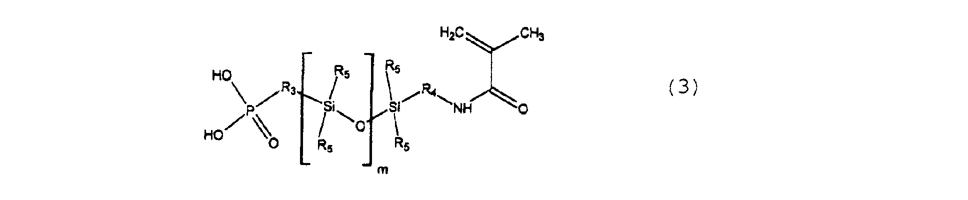

In dieser Formel sind R₃ oder R₄ Aryl- oder Alkylgruppen. Die Alkylgruppen können unverzweigt oder verzweigt sein und weisen vorzugsweise 3 bis 12 C-Atome auf. Die Substituenten R₅ sind gleich oder verschieden und stellen Methyl-, Ethyl- oder Phenylgruppen dar. m ist gleich 1 bis 10.

Unter den Phosphonsäuren mit Silikonanteilen in der Brückenverbindung zwischen reaktionsfähiger Doppelbindung und Phosphonsäure ist folgende Struktur bevorzugt:

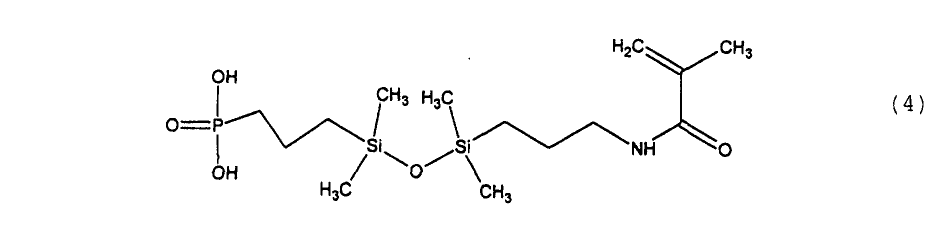

Folgende Diphosphonate sind bevorzugt in den erfindungsgemäßen Dentalmaterialien verwendbar:

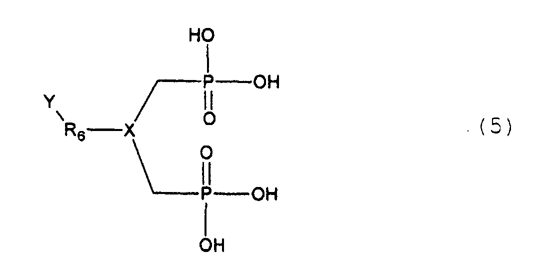

X ist Stickstoff, Bor oder eine CH-Gruppe, R₆ ist eine unverzweigte oder verzweigte Alkylgruppe mit wenigstens 3 C-Atomen, eine Arylgruppe oder eine Polyethergruppe mit 1 bis 10 Polyethereinheiten, und Y enthält ethylenisch ungesättigte Doppelbindungen, insbesondere ist Y ausgewählt aus der Gruppe bestehend aus CH₂=CH-O-, Styrol, Methacrylamid und CH₂=C(COOR₁₂)-CH₂-, wobei R₁₂ gleich H, Methyl, Ethyl, Propyl, iso-Propyl oder Butyl ist.

Gegenstand der Erfindung ist ferner die Verwendung eines erfindungsgemäßen Dentalmaterials als Haftvermittler, Compomer oder polymerisierbarer Zement.

Bei der Verwendung als Haftvermittler kann das Dentalmaterial ausschließlich aus den genannten Phosphonsäuren bzw. deren Salzen bestehen, es kann ein oder mehrere Lösungsmittel enthalten und/oder zusätzliche polymerisierbare Monomere wie insbesondere Acrylate oder Methacrylate.

Geeignete Lösungsmittel für Dentalmaterialien sind dem Fachmann geläufig, bevorzugt werden Wasser, Methanol, Ethanol, Isopropanol, Aceton, Ethylmethylketon, Ethylacetat sowie Mischungen der vorgenannten Stoffe.

Ein Zusatz wasserlöslicher Methacrylate wie z. B. Hydroxyethyl(meth)acrylate oder Hydroxypropyl(meth)acrylate ist bevorzugt. Als weitere (Meth)acrylate eignen sich bevorzugt (Meth)acrylate, die mindestens zwei Methacrylatgruppen aufweisen wie z. B. Ethylenglycoldi(meth)acrylat, Diethylenglycoldi(meth)acrylat, Triethylenglycoldi(meth)acrylat, Tetraethylenglycoldi(meth)acrylat, Hexandioldi(meth)acrylat, Butandioldi(meth)acrylat, Dodecandioldi(meth)acrylat, 2,2-Bis[p-(hydroxy(meth)acryloyloxy)phenyl]propan, ethoxyliertes Bisphenol-A-di(meth)acrylat, Glycerindi(meth)acrylat, Urethandi(meth)acrylat, Urethanpolyester-di(meth)acrylat, Trimethylolpropantri(meth)acrylat, Dipentaerytrit-penta(meth)-acrylat.

Auch eine weitere Säure in Form einer polymerisierbaren Carbonsäure kann hinzugefügt werden. Beispielhaft seien erwähnt Maleinsäure-mono-2-methacryloyloxyethylester, Phthalsäuremono-2-methacryloyloxyethylester und Trimellitsäure-mono-2-methacryloyloxyethylester.

Wenn im Rahmen der Erfindung von Dentalmaterialien die Rede ist, sind damit alle Materialien gemeint, die im Rahmen von restaurativen oder prothetischen Arbeiten an Zähnen Anwendung finden und die erfindungsgemäßen Phosphonate bzw. deren Salze enthalten. Besonders vorteilhaft sind die erfindungsgemäßen Phosphonsäuren in Haftvermittlern einsetzbar.

Erfindungsgemäße Dentalmaterialien können insbesondere folgende Inhaltsstoffe aufweisen:
- 2,5 bis 60 Gew.-% erfindungsgemäße Phosphonsäuren bzw. deren Salze,
- 5 bis 80 Gew.-% weitere radikalisch polymerisierbare Comonomere,
- 0 bis 80 Gew.-% Lösungsmittel,
- 0 bis 2 Gew.-% radikalische Polymerisationsinitiatoren,
- 0 bis 80 Gew.-% Füllstoffe (abhängig von der vorgesehenen Anwendung als Haftvermittler, Zement oder Compomer).

Für Haftvermittler ist ein Anteil von 0-20 Gew.-% Füllstoffen bevorzugt; Zemente und Compomere sind bevorzugt lösemittelfrei und der Füllstoffanteil liegt bevorzugt zwischen 40 und 80 Gew.-%.

Die Erfindung wird nachfolgend anhand von Ausführungsbeispielen beschrieben. Zunächst wird die teilweise mehrstufige Synthese erfindungsgemäßer Phosphonsäuren beschrieben, anschließend die Zusammensetzung dreier Ausführungsbeispiele erfindungsgemäßer Haftvermittler.

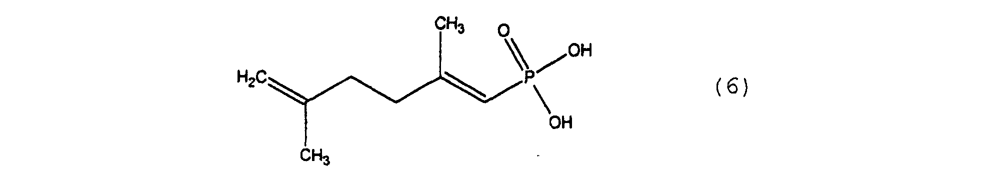

### Synthese von 1-(2,5-Dimethyl-1,5-hexadienyl)-phosphonsäure (Formel (6))

104 g (0,5 mol) PCl₅ werden in 1 l Toluol suspendiert und unter Kühlen 120 ml (0,6 mol) 2,5-Dimethyl-1,5-hexadien hinzugetropft. Man hält die Reaktion noch 3 h auf 15°C und leitet dann 3 h SO₂ bei 15°C ein. Dann werden Toluol und SOCl₂ abdestilliert, 0,5 g Triphenylphosphan hinzugefügt und die Mischung bei 180°C 8 h bei leichtem Unterdruck gehalten.
Die Mischung wird mit 100 ml Dichlormethan verdünnt und unter Kühlen und heftigem Rühren mit 300 ml einer 5 molaren NaOH-Lösung tropfenweise versetzt. Nach 2 h wird die Reaktionsmischung mit 300 ml Wasser verdünnt und unter Kühlen mit 300 ml 25 %iger Phosphorsäure versetzt.

Diese Mischung wird dreimal mit 500 ml Dichlormethan ausgeschüttelt, die organische Phase über MgSO₄ getrocknet und im Vakuum das Lösungsmittel entfernt. Es entstehen 42 g eines braunen Sirups.
Ausbeute: 44 %

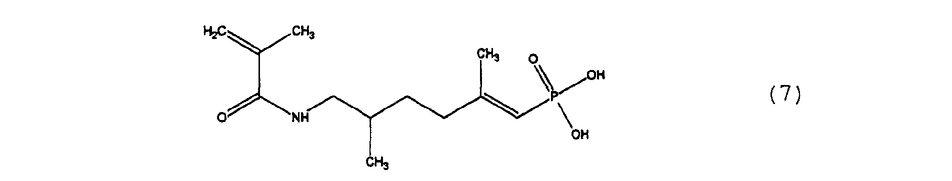

### Synthese von 6-Methacrylamido-2,5-dimethyl-1-hexenyl-phosphonsäure (Formel (7))

19 g 1-(2,5-Dimethyl-1,5-hexadienyl)-phosphonsäure (0,1 mol) werden in 100 ml Eisessig gelöst und mit 60 mg Phenothiazin und 7 g (0,1 mol) Methacrylnitril versetzt. Unter Kühlen werden 12 g 85 %iger Schwefelsäure hinzugefügt. Nach 24 h bei 50 °C wird der Ansatz gekühlt und mit 200 ml Wasser und 200 ml Dichlormethan versetzt und insgesamt dreimal mit 200 ml Dichlormethan ausgeschüttelt. Die vereinigten organischen Phasen werden noch zweimal mit Wasser ausgeschüttelt und dann über Molekularsieb getrocknet.
Ausbeute: 18 g (66 %)

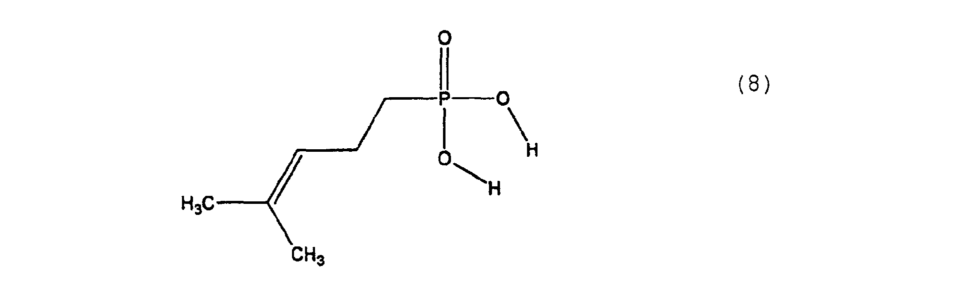

### Synthese von 1 - (4-Methyl-4-pentenyl)-phosphonsäure (Formel (8))

4 g 1-Brom-4-methyl-4-penten (48 mmol) werden 6 h mit 7,5 g (60 mmol) P(OCH₃)₃ bei 120°C unter Stickstoff erhitzt. Danach wird die Lösung auf Raumtemperatur gebracht und mit 35 ml 37 %iger Salzsäure versetzt und nochmals 20 h auf 100°C erhitzt. Danach wird die Lösung wieder abgekühlt und mit 10 ml Wasser versetzt. Die wässrige Phase wird isoliert, es bildet sich ein leicht bräunlicher Niederschlag, der an der Luft getrocknet wird.
Ausbeute: 5,6 g 71 %

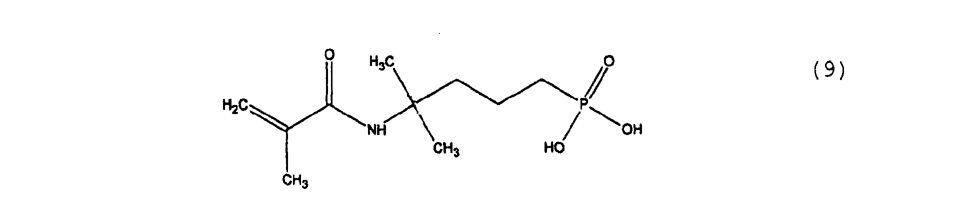

### Synthese von 4-Methacrylamido-4-methyl-pentyl-phosphonsäure (Formel (9))

1,9 g 1-(4-Methyl-4-pentenyl)-phosphonsäure (12) (10 mmol) werden in 10 ml Eisessig gelöst und mit 6 mg Phenothiazin und 0,7 g (10 mmol) Methacrylnitril versetzt. Unter Kühlen werden 1,2 g 85 %iger Schwefelsäure hinzugefügt. Nach 24 h bei 50°C wird der Ansatz gekühlt und mit 20 ml Wasser und 20 ml Dichlormethan versetzt und insgesamt dreimal mit 20 ml Dichlormethan ausgeschüttelt. Die vereinigten organischen Phasen werden noch zweimal mit Wasser ausgeschüttelt und dann über Molekularsieb getrocknet.
Ausbeute: 1,3 g (52 %)

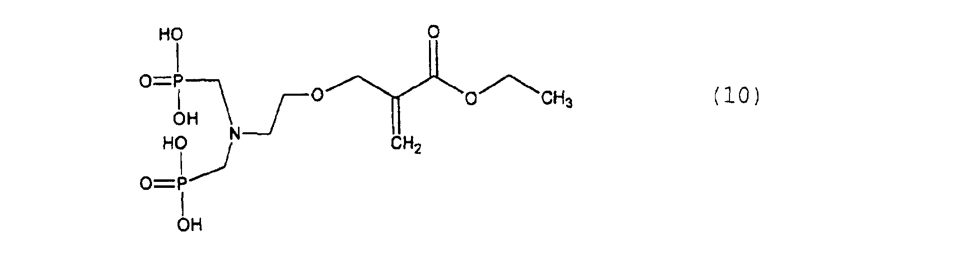

### Synthese von 2-(Ethyloxycarbonyl)-2-propenyl-1-oxyethyl-aminobismethylenphosphonsäure (Formel (10))

4,45 g (60 mmol) KOH werden in 50 ml DMSO fünf Minuten bei Raumtemperatur gerührt. Dann werden 2,49 g (10 mmol) Ethanol-amino-N,N-bismethylenphosponsäure vorsichtig zugegeben. Zu der schwach gefärbten Suspension werden langsam 2,94 g (15 mmol) α-Brommethylacrylsäureethylester zugetropft. Der Reaktionsansatz wird 24 h bei Raumtemperatur gerührt, anschließend mit 50 ml kaltem Wasser versetzt, auf einen sauren pH eingestellt, mit 50 ml Dichlormethan extrahiert, die organische Phase getrocknet und das Lösungsmittel im Vakuum abgetrennt. Der verbleibende wachsartige Rückstand wird im Trokkenschrank getrocknet.
Ausbeute: 1,5 g (40 %)

Die nachfolgende Tabelle zeigt die Zusammensetzung von drei erfindungsgemäßen Haftvermittlern (Beispiele 1 - 3)

| | Beispiel 1 | Beispiel 2 | Beispiel 3 |
|---|---|---|---|
| Phosphonat | 10 g | 1 g | 1 g |
| | Formel (7) | Formel (9) | Formel (10) |
| H₂O | 5 g | 1 g | 1,5 g |
| Ethanol | 20 g | 4,5 g | 10 g |
| Bis-GMA* | 25 g | 0,75 g | 1 g |
| TEDMA** | 25 g | 1,25 g | 5 g |
| HEMA*** | 15 g | 1,5 g | 1,5 g |
| Ethyl-dimethylamino-benzoat | 0,15 g | 0,018 g | 0,018 g |
| Campherchinon | 0,1 g | 0,014 g | 0,014 g |

| | | | |
|---|---|---|---|
| * Isopropyliden bis[2-hydroxy-3-(4-phenoxy)-methacrylat] | | | |
| ** Triethylenglycoldimethacrylat | | | |
| *** 2-Hydroxyethylmethacrylat | | | |

## Patentansprüche

1. Dentalmaterial, **dadurch gekennzeichnet, daß** es eine oder mehrere Phosphonsäuren folgender Struktur und/oder Salze dieser Säuren enthält: 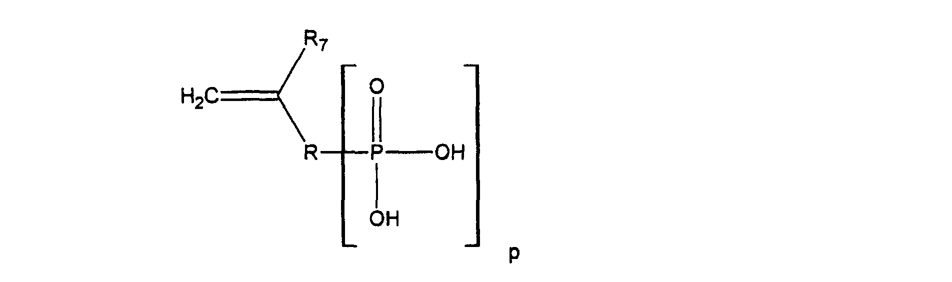 wobei R, R₇ und p unabhängig voneinander bedeuten:
entweder:
a) R: Alkylgruppe oder Alkylengruppe mit mindestens 6 C-Atomen oder Arylgruppe,
R₇: H, Methyl, Ethyl, Propyl, Isopropyl oder Butyl, p gleich 1 oder 2;
oder:
b) R: -CO-NR₁-R₈-
mit R₁ gleich H, Alkyl oder Aryl,
mit R₈ gleich Aryl oder CₙH₂ₙ
wobei 4 ≤ n ≤ 18 ist,
oder R₈ gleich CₙH₂ₙ-Si(R₅)₂-[O-Si(R₅)₂]ₘ-CₙH₂ₙ-
wobei 3 ≤ n ≤ 12,
1 ≤ m ≤ 10, und
R₅ gleich Methyl, Ethyl oder Phenyl ist,
oder R₈ gleich CₙH₂ₙ-COONH-CₙH₂ₙ
wobei 4 ≤ n ≤ 12 ist, und wobei R₈ ether- oder weitere Urethangruppen aufweisen kann,
R₇ und p wie in a) definiert sind;
oder:
c) R: 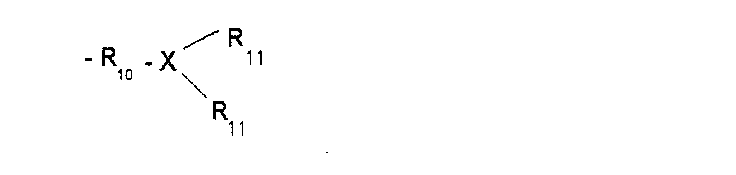 mit R₁₀ ausgewählt aus der Gruppe bestehend aus Arylgruppen, Alkylgruppen mit mindestens 3 C-Atomen, oder Polyethergruppen mit 1 bis 10 Polyethereinheiten, mit R₁₁ gleich oder verschieden ausgewählt aus der Gruppe bestehend aus Alkylgruppen und Arylgruppen, mit X gleich N, B oder CH,
R₇: COOR₉, CONHR₉, H oder Phenyl mit R₉ ausgewählt aus der Gruppe bestehend aus Methyl, Ethyl, Propyl, Iso-propyl und Butyl,
p gleich 2.

2. Dentalmaterial nach Anspruch 1, **dadurch gekennzeichnet, daß** die Phosphonsäuren folgende Struktur aufweisen: 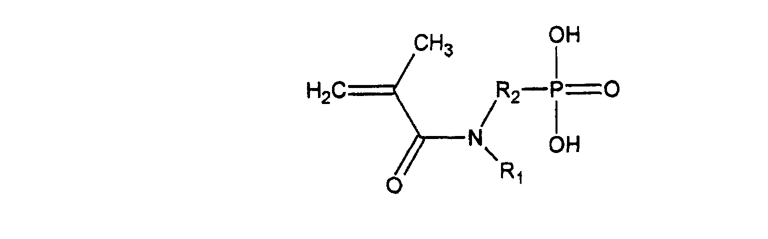 wobei R₂ eine Alkylgruppe mit mindestens 4, vorzugsweise 6 bis 12 C-Atomen oder eine Arylgruppe und R₁ gleich Alkyl, Aryl oder H ist.

3. Dentalmaterial nach Anspruch 1, **dadurch gekennzeichnet, daß** die Phosphonsäuren folgende Struktur aufweisen: 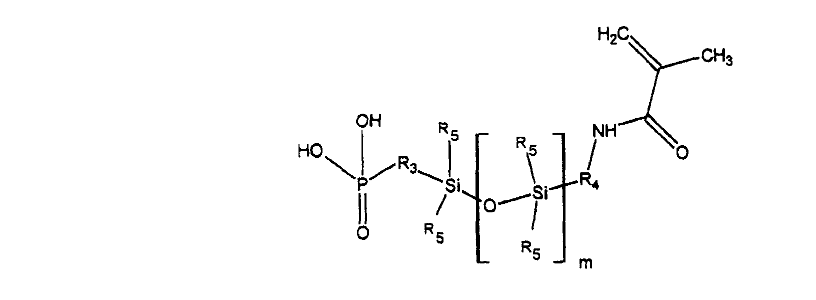 wobei R₃, R₄ gleich oder verschieden Aryl oder Alkyl darstellt, R₅ gleich oder verschieden Methyl, Ethyl oder Phenyl darstellt, und m gleich 1 bis 10 ist.

4. Dentalmaterial nach Anspruch 1, **dadurch gekennzeichnet, daß** die Phosphonsäuren folgende Struktur aufweisen: 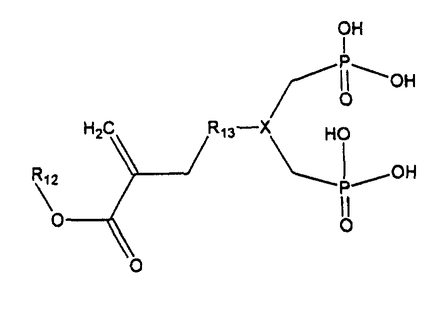 wobei bedeuten:
X gleich N, B, oder CH,
R₁₂ gleich H, Methyl, Ethyl, Propyl, iso-Propyl oder Butyl,
R₁₃ gleich Alkyl mit mindestens 3 C-Atomen Kettenlänge, Aryl oder eine Polyethergruppe mit 1 bis 10 Polyether-Einheiten.

5. Dentalmaterial nach einem der Ansprüche 1 bis 4, , **dadurch gekennzeichnet, daß** es zusätzlich Lösungsmittel enthält.

6. Dentalmaterial nach Anspruch 5, **dadurch gekennzeichnet, daß** das Lösungsmittel ausgewählt ist aus der Gruppe bestehend aus Wasser, Ethanol, Methanol, Isopropanol, Aceton, Ethylmethylketon und Ethylacetat.

7. Dentalmaterial nach einem der Ansprüche 1 bis 6, **dadurch gekennzeichnet, daß** es Erdalkali-, Alkali- und/oder Ammonium-Salz der Phosphonsäuren enthält.

8. Dentalmaterial nach einem der Ansprüche 1 bis 7, **dadurch gekennzeichnet, daß** es zusätzlich Mono-, Di- oder Oligomethacrylate enthält.

9. Dentalmaterial nach einem der Ansprüche 1 bis 8, **dadurch gekennzeichnet, daß** es zusätzlich Füllstoffe enthält.

10. Dentalmaterial nach Anspruch 9, **dadurch gekennzeichnet, daß** die Füllstoffe ionenfreisetzend sind.

11. Dentalmaterial nach einem der Ansprüche 1 bis 10, **dadurch gekennzeichnet, daß** es zusätzlich Starter enthält.

12. Verwendung eines Dentalmaterials nach einem der Ansprüche 1 bis 11 als Haftvermittler oder Bestandteil eines Haftvermittlers.

13. Verwendung eines Dentalmaterials nach einem der Ansprüche 1 bis 11 als Compomer oder polymerisierbarer Zement oder als Bestandteil eines solchen Compomers bzw. Zements.
